Europäisches Patentamt

European Patent Office

Office européen des brevets

(19)

(11) Veröffentlichungsnummer: **0 162 252**
**B1**

(12) # EUROPÄISCHE PATENTSCHRIFT

(45) Veröffentlichungstag der Patentschrift:
**18.01.89**

(51) Int. Cl.⁴: **C 07 D 323/06** // B01D9/02

(21) Anmeldenummer: **85104100.4**

(22) Anmeldetag: **04.04.85**

(54) **Verfahren zur Herstellung von reinem Trioxan.**

(30) Priorität: **13.04.84 DE 3413903**
**12.03.85 DE 3508668**

(43) Veröffentlichungstag der Anmeldung:
**27.11.85 Patentblatt 85/48**

(45) Bekanntmachung des Hinweises auf die Patenterteilung:
**18.01.89 Patentblatt 89/3**

(84) Benannte Vertragsstaaten:
**AT BE CH DE FR GB IT LI NL SE**

(56) Entgegenhaltungen:
**DE-A- 2 855 710**
**DE-B- 1 643 414**

(73) Patentinhaber: **HOECHST AKTIENGESELLSCHAFT,
Postfach 80 03 20, D-6230 Frankfurt am Main 80 (DE)**

(72) Erfinder: **Rittner, Siegbert, Dr., Kornblumenweg 5,
D-6105 Mörfelden-Walldorf (DE)**
Erfinder: **Burg, Karlheinz, Dr., Eichenweg 18,
D-6200 Wiesbaden (DE)**
Erfinder: **Schlaf, Helmut, Dr., Zum Gimbacher Hof 20,
D-6233 Kelkheim (Taunus) (DE)**

## Beschreibung

Nach dem Stand der Technik wird Trioxan für gewöhnlich durch Erhitzen von 30–70%igen wässrigen Formaldehydlösungen in Gegenwart von sauren Katalysatoren, z.B. 2–25% Mineralsäure (DE-Patentschrift 1 543 390), oder in Gegenwart von sauren Ionenaustauschern (DE-Patentschrift 1 135 491) hergestellt. Das Trioxan wird destillativ aus dem Reaktionsgemisch entfernt. Dies geschieht entweder in einer vom Reaktor aufgesetzten Kolonne gemäss der US-Patentschrift 2 304 080 oder in einer separaten Kolonne, wie in der GB-Patentschrift 1 012 372 beschrieben. Das trioxanreiche, basenhaltige Destillat wird mit Methylenchlorid, Ethylenchlorid, Benzol oder Toluol extrahiert wie z.B. in der DE-Auslegeschrift 1 668 687 angegeben. Anschliessend wird der Extrakt neutralisiert und destillativ gereinigt, wie beispielsweise in der DE-Patentschrift 1 543 815 beschrieben.

Die erwähnte Extraktion ist notwendig, um die Abtrennung des Trioxans vom Wasser zu erreichen, während die genannte destillative Reinigung erforderlich ist, um bei der Trioxansynthese entstehende Nebenprodukte, wie Formaldehyd, Methanol, Ameisensäure, Methylformiat, Methylal, Dioxymethylendimethylether u.a. zu entfernen und so zu einem polymerisationsfähigen Trioxan zu kommen. Bei diesen Destillationsschritten werden auch höher siedende Verunreinigungen entfernt, die im wieder aufzuarbeitenden, bei der Polymerisation nicht umgesetzten Trioxan enthalten sind.

Für die genannte Endreinigung sind auch kombinierte Reinigungsverfahren vorgeschlagen worden. So ist aus der DE-Patentschrift 1 237 583 bekannt, vor der fraktionierten Destillation das geschmolzene Trioxan mit schwach sauren Ionenaustauschern zu behandeln, wodurch eine weitgehende Abtrennung der basisch wirkenden Verunreinigungen erreicht werden soll. Spezielle Ionenaustauscher zur Reinigung von Trioxan sind Gegenstand der DE-Offenlegungsschrift 2 156 112.

Aus der US-Patentschrift 3 281 336 ist bekannt, die Abtrennung von Verunreinigungen im Trioxan durch Extraktivdestillation in Gegenwart von Wasser oder Ethylenglykolen zu erreichen.

Neben einer Reihe von weiteren Verfahren zur Reinigung von Trioxan, wie der Verwendung von aktiviertem Aluminiumoxid oder einem Molekularsieb, ist aus der DE-Offenlegungsschrift 2 855 710 bekannt, zur Abtrennung der Verunreinigungen flüssiges Trioxan in einem offenen System zu kristallisieren und Luft oder ein Inertgas evtl. unter Druck darüber zu bewegen. Durch Überleiten beispielsweise von Luft können aber neue Verunreinigungen im Trioxan gebildet werden. Ausserdem werden nach dieser Verfahrensweise überwiegend nur die leicht siedenden Verunreinigungen entfernt. Schwersieder, welche die Polymerisation ebenfalls stören können, verbleiben noch in signifikativen Mengen im Produkt. Nachteilig wirkt sich auch der hohe Sublimationsdruck des Trioxans bei dem geschilderten Verfahren in der Weise aus, dass Materialverluste entstehen.

Generell kann man von den bekannten Verfahren zur Aufarbeitung von Trioxangemischen sagen, dass sie energieintensiv und apparativ aufwendig sind, dass durch Einsatz zusätzlicher Hilfsstoffe unvorhersehbare Nebenreaktionen zu befürchten sind und dass sie teilweise nicht zu einem Trioxan mit der erforderlichen Reinheit führen.

Die Aufgabe vorliegender Erfindung bestand daher darin, ein Verfahren zur Aufarbeitung von Trioxangemischen bereitzustellen, das die Nachteile des Standes der Technik nicht besitzt und das insbesondere bei einer apparativ einfachen und weniger energieintensiven Verfahrensweise zu einem hochreinen Trioxan führt.

Diese Aufgabe wird erfindungsgemäss in der Weise gelöst, dass das Trioxangemisch solange einer vorzugsweise mehrstufigen fraktionierten Kristallisation in einem geschlossenen System unterworfen wird, bis ein Trioxan der erforderlichen Reinheit anfällt.

Die Erfindung betrifft daher ein Verfahren zur Herstellung von hochreinem Trioxan durch Kristallisation eines Trioxangemisches, dadurch gekennzeichnet, dass das Trioxangemisch in einem geschlossenen System einer ein- oder mehrstufigen Kristallisation unterworfen wird, wobei die letzte Stufe der Kristallisation als Schmelzkristallisation erfolgt.

Bevorzugt wird das erfindungsgemässe Verfahren unter Inertgasatmosphäre und zumindest in den letzten Stufen, vorzugsweise in allen Stufen der Schmelzkristallisation, unter weitgehendem Feuchtigkeitsausschluss durchgeführt. Als Inertgas kommt dabei vor allem Stickstoff in Frage. Daneben können für diesen Zweck auch Kohlendioxid oder Argon eingesetzt werden.

Unter «Trioxangemisch» sei hierbei sowohl das bei der bekannten Trioxansynthese anfallende wässrige System als auch das bereits vorgereinigte und dann weitgehend wasserfreie, im allgemeinen weniger als 20 Gew.%, vorzugsweise weniger als 10 Gew.% und insbesondere weniger als 1 Gew.% Wasser enthaltende Gemisch verstanden. Weiterhin umfasst dieser Begriff auch das Trioxan, das bei der Polymerisation zu Polyoxymethylen nicht umgesetzt wird und das nach entsprechender Aufarbeitung wieder in dem Polymerisationsprozess eingesetzt werden soll. Da das Trioxan mit den vorhandenen Verunreinigungen ein eutektisches System bildet, sind für die fraktionierte Schmelzkristallisation grundsätzlich alle jene Trioxangemische geeignet, deren Trioxangehalte oberhalb der eutektischen Zusammensetzung liegen. Als Begleitstoffe enthalten diese Trioxangemische Verbindungen wie beispielsweise Wasser, Ameisensäure, Formaldehyd, Methanol, Methylal, Dioxymethylendimethylether, Dioxolan, Dioxan, Trioxepan, Trioxymethylendimethylether, Tetroxan udgl. In der Regel beträgt der Trioxangehalt des als Ausgangsgemisch für die Schmelzkristallisation eingesetzten Trioxangemisches bei Beginn der Reinigung mindestens 50 Gew.%, insbesondere mindestens 90 Gew.%, und besonders bevorzugt mindestens 95 Gew.%. Grundsätzlich kann der Trioxangehalt auch unterhalb von 50 Gew.% und damit noch näher am Eutektikum liegen. Jedoch lässt sich dann nur noch wenig reines Trioxan abscheiden.

Falls das für die erfindungsgemässe Aufbereitung eingesetzte Trioxangemisch wasserhaltig ist, sollte der Trioxangehalt in der Regel mindestens 20 Gew.%, vorzugsweise mindestens 50 Gew.% betragen, da ansonsten die in die Kristallisationsapparatur eingebrachten Wassermengen recht gross und die abgeschiedene Trioxanmenge recht klein wird bzw. die erforderliche Kristallisationstemperatur recht tief liegt. In Abhängigkeit von der Wassermenge und der Temperatur des wasserhaltigen Trioxangemisches läuft die erste Stufe überwiegend als Schmelzkristallisation oder als Lösungskristallisation (d.h. als kristalline Abscheidung aus der Lösung) ab, wobei auch im letzteren Fall diese Lösungskristallisation und die nachfolgende Schmelzkristallisation erfindungsgemäss in der gleichen Apparatur erfolgen. Eine Lösungskristallisation findet in der ersten Stufe beispielsweise dann statt, wenn bei Temperaturen von etwa 60°C bis etwa 70°C die Wassermenge des Trioxangemisches mindestens etwa 20 Gew.% beträgt. Das nach der Lösungskristallisation für die Schmelzkristallisation anfallende Trioxangemisch ist im allgemeinen bereits nach der ersten Stufe weitgehend wasserfrei und enthält mindestens 50 Gew.% Trioxan.

Die bei dem erfindungsgemässen Verfahren angewandten Kristallisationstemperaturen können in weiten Grenzen schwanken und liegen im allgemeinen im Bereich vom −10 bis +63°C, vorzugsweise zwischen +5 und +63°C. Zur schnellen Auslösung der Kristallisation ist der Einsatz von Impfkristallen empfehlenswert. Die günstigsten Temperaturen für die Auslösung der Kristallisation und die günstigsten Kristallisationszeiten lassen sich experimentell leicht ermitteln. Die Art der dabei gebildeten Kristalle ist erfindungsgemäss nicht kritisch. Nach Kristallisation des aufgeschmolzenen Rohtrioxans wird der verbliebene Flüssiganteil rasch entfernt und das erhaltene, schon reinere Trioxan nach Aufschmelzen in analoger Weise vorzugsweise noch ein- oder mehrmal der Schmelzkristallisation unterworfen, bis der erforderliche Reinheitsgrad erreicht ist. Zumeist ist dies bereits bei zwei oder drei Stufen der Fall. Der in den Kristallisationsstufen anfallende, nichtkristallisierte Flüssiganteil, der noch Trioxan enthält, kann immer wieder zurückgeführt und erneut der Kristallisation unterworfen werden. Dies ist theoretisch so lange möglich, bis die eutektische Zusammensetzung im Flüssiganteil erreicht ist. Für praktische Zwecke wird man einige Prozent oberhalb dieser eutektischen Zusammensetzung bleiben. In gleicher Weise kann die bei der Lösungskristallisation anfallende, noch trioxanhaltige, wässrige Mutterlauge zurückgeführt und bei tieferer Temperatur einer erneuten Kristallisation unterworfen werden. Auch dieses Prozedere wird man aus wirtschaftlichen Gründen im allgemeinen nur bis zu einem bestimmten Trioxanrestgehalt in der Lösung wiederholen.

Das erfindungsgemässe Verfahren kann prinzipiell in jedem geschlossenen Kristallisator durchgeführt werden, der eine vorzugsweise mehrstufige und in schneller Folge ablaufende Schmelzkristallisation, vorzugsweise unter Inertgas und Feuchtigkeitsausschluss erlaubt. Hierfür kommt z.B. ein Röhrenkristallisator (sogenannter Tropfapparat) in Frage, wie er in Winnacker-Küchler, Chemische Technologie, 4. Aufl., Bd. 6 (1982), S. 148, beschrieben ist und der diskontinuierlich betrieben wird. Bevorzugt wird erfindungsgemäss ein Kristallisator eingesetzt, wie er in der US-Patentschrift 3 621 664 beschrieben ist und der eine semikontinuierliche Verfahrensführung erlaubt. Dieser Kristallisator besteht im Prinzip aus einer vorzugsweise grösseren Zahl von parallel geschalteten, zumeist senkrecht angeordneten Kristallisationselementen(röhren), die das zu trennende, flüssige Gemisch als Rieselfilm durchläuft. An den gekühlten Wandungen erfolgt dann die Kristallisation. Nach Erreichen einer bestimmten Dicke der Kristallschicht wird die Zufuhr dès flüssigen Gemisches unterbrochen, der an der Kristallschicht anhaftende Flüssiganteil entfernt und anschliessend die Kristallschicht abgeschmolzen.

Das nach dem erfindungsgemässen Verfahren gereinigte Trioxan besitzt im allgemeinen eine Reinheit von 99,99%, vorzugsweise 99,995%, und liefert bei der Homo- oder Copolymerisation Polymerisate mit hohem Molekulargewicht und ausgezeichneter Wärmebeständigkeit.

Neben der bevorzugten Verwendung als Monomeres für Polymere und Copolymere kann das erfindungsgemäss erhaltene Trioxan auch als Synthesebaustein, z.B. für Pharmazeutika, Pflanzenschutz, Textilhilfsmittel und Konservierungsmittel, ggf. in Gegenwart eines Stabilisators, eingesetzt werden.

Die Vorteile des erfindungsgemässen Verfahrens liegen nicht nur in der sehr hohen Reinheit des erhaltenen Trioxans, seiner produktschonenden und umweltfreundlichen Arbeitsweise, sondern auch in der beträchtlichen Energieersparnis, welches das Verfahren gegenüber der mehrstufigen fraktionierten Destillation bietet.

Es war nicht zu erwarten gewesen und ist als überraschend anzusehen, dass das Trioxan auch in reinem und reinstem Zustand ein mehrmaliges Aufschmelzen und Erstarren übersteht, ohne dass eine Spontanpolymerisation einsetzt. Weiterhin war nicht vorhersehbar, dass sich bei wasserhaltigen Trioxangemischen die Lösungskristallisation und die Schmelzkristallisation in der gleichen Apparatur durchführen lassen.

Das erfindungsgemässe Verfahren wird anhand der folgenden Beispiele erläutert, ohne es auf diese Beispiele einzuschränken. Die Prozentzahlen sind jeweils Gewichtsprozente.

## Beispiel 1

In einem feuchtefreien und mit Stickstoff inertisierten Röhrenkristallisator (wie in Winnacker-Küchler, Chemische Technologie, 4. Aufl., Bd. 6 (1982), S. 148 beschrieben), dessen Mantel an einen Thermostaten mit einem Temperatur-Zeitplangeber angeschlossen war, wurde ein destillativ aufkonzentriertes Trioxan (aus der Trioxansynthese aus wässrigen Formaldehydlösungen in Gegenwart saurer Katalysatoren, Trioxangehalt: ca. 94,5%) mit einem Erstarrungspunkt von +58,5°C und folgender Zusammensetzung eingesetzt:

| Methylal | Methanol | Dioxy-ether * | Dioxan | Trioxepan | Trioxy-ether | Dimethyl-bicyclo-tetroxan | Methyl-dicyclo-tetroxan | Bicyclo-tetroxan | Tetroxan |
|---|---|---|---|---|---|---|---|---|---|
| – | 2,7 ppm | 4,3% | 0,15% | 0,25% | 0,71% | 30 ppm | 600 ppm | 0,13% | 80 ppm |

*Dioxyether = Dioxymethylendimethylether

Das Rohtrioxan wurde unter Stickstoffüberlagerung auf 58°C abgekühlt und mit wenigen Impfkristallen angeimpft. Nach etwa 30 Minuten wurde der Kristallisatorinhalt innerhalb von einer Stunde auf 50°C abgekühlt und der im Apparat verbliebene Flüssiganteil rasch abgelassen. Die im Apparat verbliebenen gut ausgebildeten Kristalle wurden dann unter Stickstoff aufgeschmolzen, die Schmelze, die nun einen Erstarrungspunkt von 60°C hatte, bei dieser Temperatur mit Impfkristallen angeimpft und einer erneuten, unter ähnlichen Temperatur- und Zeitbedingungen ablaufenden Schmelzkristallisation unterworfen. Nach Abtrennung des Flüssiganteils der 2. Stufe wurden die im Apparat zurückgebliebenen Kristalle erneut aufgeschmolzen und die Schmelze unter Stickstoff- und Feuchtigkeitsausschluss isoliert. Pro 100 Gew.-Teile zweistufig kristallisierten Trioxans wurden 130 Gew.-Teile Rohtrioxan verwendet. Das isolierte Trioxan hatte die folgenden Restverunreinigungen:

| Methylal | Methanol | Dioxy-ether | Dioxan | Trioxepan | Trioxy-ether | Dimethyl-bicyclo-tetroxan | Methyl-dicyclo-tetroxan | Bicyclo-tetroxan | Tetroxan |
|---|---|---|---|---|---|---|---|---|---|
| — | — | 35 ppm | — | — | — | — | — | — | — |

## Beispiel 2

In einem Kristallisator wie in der US-Patentschrift 3 621 664 beschrieben, wurde eine wässrige Lösung mit 55 Gew.% Trioxan bei 70°C vorgelegt. Durch stufenweises Absenken der Temperatur über einen Zeitraum von 35 min auf +10° wurde das Trioxan kristallisiert. Der Rückstand, der noch 12 Gew.% Trioxan enthielt, wurde aus dem System entfernt. Durch zweimaliges partielles Abschmelzen von je 800 g des ankristallisierten Trioxans durch stufenweises Anheben der Temperatur auf 55°C wurde ein Trioxan mit 95 proz. Reinheit erhalten. Zur weiteren Reinigung wurde das Trioxan einer mehrstufigen Schmelzkristallisation entsprechend Beispiel 3 unterworfen.

## Beispiel 3

In einem Kristallisator, wie in der US-Patentschrift 3 621 664 beschrieben, wurde in feuchtefreier und mit Stickstoff gut inertisierter Atmosphäre ein destillativ aufkonzentriertes Trioxan (aus der Trioxansynthese aus wässrigen Formaldehydlösungen in Gegenwart saurer Katalysatoren, Trioxangehalt: ca. 97,5%) der folgenden Zusammensetzung zur Schmelzkristallisation vorgelegt:

| Methylal | Methanol | Dioxy-ether | Dioxan | Trioxepan | Trioxy-ether | Dimethyl-bicyclo-tetroxan | Methyl-dicyclo-tetroxan | Bicyclo-tetroxan | Tetroxan |
|---|---|---|---|---|---|---|---|---|---|
| <10 ppm | 96 ppm | 1,7% | 970 ppm | 0,18% | 0,17% | 40 ppm | 500 ppm | 0,21% | 200 ppm |

Es wurde dreistufig in einem Temperaturbereich von +10°C bis +63°C kristallisiert, wobei nach 140 Minuten Gesamtkristallisations- und Aufschmelzzeit das geschmolzene Kristallisat der dritten Stufe direkt zur Polymerisation verwendet wurde. Pro 100 Gew.-Teile dreistufig gereinigten Trioxans wurden 106 Gew.-Teile destillativ vorgereinigtes Trioxan benötigt. Im dreistufig gereinigten Trioxan konnte gaschromatografisch keine der im Ausgangsprodukt enthaltenen Verunreinigungen mehr nachgewiesen werden.

## Beispiel 4

Im gleichen Kristallisator und unter gleichen Bedingungen wie im Beispiel 3 beschrieben wurde ein destillativ aufkonzentriertes Trioxan (Trioxangehalt: ca. 97%) der folgenden Zusammensetzung zur Schmelzkristallisation vorgelegt:

| Methylal | Methanol | Dioxy-ether | Dioxan | Trioxepan | Trioxy-ether | Dimethyl-bicyclo-tetroxan | Methyl-dicyclo-tetroxan | Bicyclo-tetroxan | Tetroxan |
|---|---|---|---|---|---|---|---|---|---|
| <10 ppm | 197 ppm | 2,4% | 0,12% | 0,22% | 0,22% | 20 ppm | 400 ppm | 0,17% | 100 ppm |

Man kristallisierte vierstufig in einem Temperaturbereich von +5°C bis +63°C, wobei nach 160 Minuten Gesamtkristallisations- und Aufschmelzzeit das geschmolzene Kristallisat der vierten Stufe direkt zur Polymerisation eingesetzt wurde. Pro 100 Gew.-Teile vierstufig gereinigten Trioxans wurden 108 Gew.-Teile destillativ vorgereinigtes Trioxan benötigt. Nicht nur nach der vierten, sondern bereits auch schon nach der dritten Kristallisationsstufe konnte keine der im Ausgangsprodukt enthaltenen Verunreinigungen gaschromatografisch mehr nachgewiesen werden.

## Patentansprüche

1. Verfahren zur Herstellung von hochreinem Trioxan durch Kristallisation eines Trioxangemisches, dadurch gekennzeichnet, dass das Trioxangemisch in einem geschlossenen System einer ein- oder mehrstufigen Kristallisation unterworfen wird, wobei die letzte Stufe der Kristallisation als Schmelzkristallisation erfolgt.

2. Verfahren nach Anspruch 1, dadurch gekennzeichnet, dass die Kristallisation mehrstufig, in Gegenwart eines Inertgases und zumindest in den letzten Stufen unter Feuchtigkeitsausschluss erfolgt.

3. Verfahren nach Anspruch 1 oder 2, dadurch gekennzeichnet, dass das Trioxangemisch mindestens 50 Gew.-% Trioxan enthält.

4. Verfahren nach einem oder mehreren der Ansprüche 1 bis 3, dadurch gekennzeichnet, dass das Trioxangemisch weitgehend wasserfrei ist und die Kristallisation nur als Schmelzkristallisation erfolgt.

5. Verfahren nach einem oder mehreren der Ansprüche 1, 2 und 3, dadurch gekennzeichnet, dass das Trioxangemisch 30 bis 50 Gew.-% Wasser enthält und die erste Stufe der Kristallisation als Lösungskristallisation erfolgt.

6. Verfahren nach einem oder mehreren der Ansprüche 1 bis 5, dadurch gekennzeichnet, dass die Kristallisationstemperatur zwischen +5 und +63°C liegt.

## Claims

1. A process for the preparation of highly pure trioxane by crystallization of a trioxane mixture, which comprises the trioxane mixture being subjected, in a closed system, to a one-step or multistep crystallization, with the last step of the crystallization being carried out as melt crystallization.

2. The process as claimed in claim 1, wehrein the crystallization is carried out in several steps, in the presence of an inert gas and, at least in the last steps, with exclusion of moisture.

3. The process as claimed in claim 1 or 2, wherein the trioxane mixture contains at least 50% by weight of trioxane.

4. The process as claimed in one or more of claims 1 to 3, wherein the trioxane mixture ist substantially anhydrous, and the crystallization is carried out only as melt crystallization.

5. The process as claimed in one or more of claims 1, 2 and 3, wherein the trioxane mixture contains 30 to 50% by weight of water, and the first step of the crystallization is carried out as solution crystallization.

6. The process as claimed in one or more of claims 1 to 5, wherein the crystallization temperature is between +5 and +63°C.

## Revendications

1. Procédé pour préparer du trioxanne extrêment pur par cristallisation d'un mélange à base de trioxanne, procédé caractérisé en ce qu'on soumet le mélange à base de trioxanne, dans un système fermé, à une cristallisation à une ou à plusieurs étapes, la dernière étape de cristallisation étant exécutée sous la forme d'une cristallisation à partir de l'état fondu.

2. Procédé selon la revendication 1 caractérisé en ce que la cristallisation est effectuée en plusieurs étapes, en présence d'un gaz inerte et, au moins dans les dernières étapes, à l'abri de l'humidité.

3. Procédé selon l'une des revendications 1 et 2, caractérisé en ce que le mélange à base de trioxanne contient au moins 50% en poids de trioxanne.

4. Procédé selon l'une quelconque des revendications 1 à 3, caractérisé en ce que le mélange à base de trioxanne est pratiquement anhydre et en ce que la cristallisation est exécutée uniquement sous la forme d'une cristallisation à partir de l'état fondu.

5. Procédé selon l'une quelconque des revendications 1 à 3, caractérisé en ce que le mélange à base de trioxanne contient de 30 à 50% en poids d'eau et en ce que la première étape de la cristallisation est exécutée sous la forme d'une cristallisation à partir de l'état dissous.

6. Procédé selon l'une quelconque des revendications 1 à 5, caractérisé en ce que la température de cristallisation est comprise entre +5 et +63°C.